(19) Europäisches Patentamt
European Patent Office
Office européen des brevets

(11) Publication number: **0 255 278 B1**

(12) **EUROPEAN PATENT SPECIFICATION**

(45) Date of publication of patent specification: **11.03.92**

(51) Int. Cl.[5]: **C07F 7/18**, C07D 205/08, C07D 499/00

(21) Application number: **87306402.6**

(22) Date of filing: **20.07.87**

# (54) Synthesis of azetidinones.

(30) Priority: **21.07.86 US 887394**

(43) Date of publication of application:
**03.02.88 Bulletin 88/05**

(45) Publication of the grant of the patent:
**11.03.92 Bulletin 92/11**

(84) Designated Contracting States:
**AT BE CH DE ES FR GB GR IT LI LU NL SE**

(56) References cited:
**EP-A- 0 058 317**
**EP-A- 0 080 162**
**EP-A- 0 110 280**
**EP-A- 0 115 308**
**GB-A- 2 156 814**

**JOURNAL OF ORGANIC CHEMISTRY, vol. 51, no. 23, 1986, pages 4488-4490, American Chemical Society; D. GALA et al.: "Tetrahydropyran as an efficient alcohol protecting group for the synthesis of penems: synthesis of Sch 34343"**

(73) Proprietor: **SCHERING CORPORATION**
**2000 Galloping Hill Road**
**Kenilworth New Jersey 07033(US)**

(72) Inventor: **Hou, Donald Chen-Tung**
**13 Nassau Road**
**Verono New Jersey 07044(US)**
Inventor: **Wong, Yee-Shing**
**248 Getty Avenue**
**Clifton New Jersey 07011(US)**
Inventor: **Gala, Dinesh**
**9 Silvester Court**
**East Brunswick New Jersey 08816(US)**
Inventor: **Steinman, Martin**
**46 Glendale Avenue**
**Livingston New Jersey 07039(US)**

(74) Representative: **Ritter, Stephen David et al**
**Mathys & Squire 10 Fleet Street**
**London EC4Y 1AY(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to an improvement in a multi-step stereospecific process for producing azetidinones which are useful as intermediates for preparing penem antibiotics. More particularly this invention relates to an improvement in a stereospecific multi-step process in which a compound of the formula

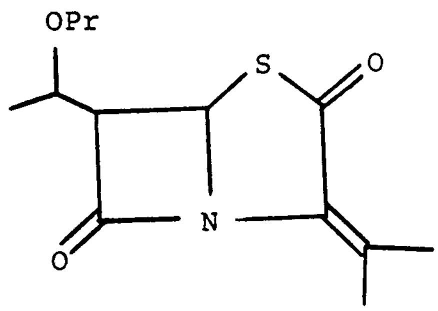

10

wherein Pr is hydrogen or a hydroxy protecting group is converted to a compound of the formula

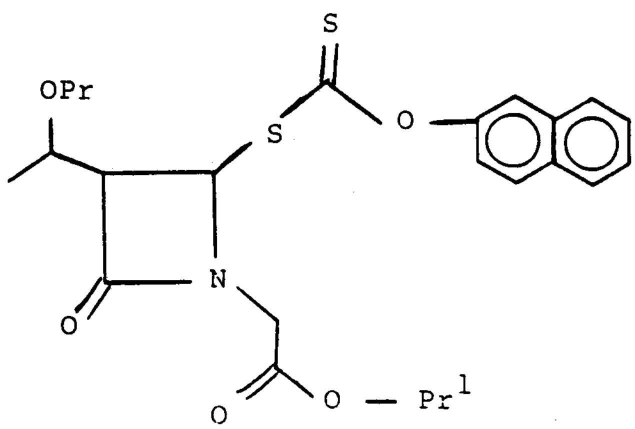

I

wherein Pr is hydrogen or a hydroxy protecting group and $Pr^1$ is a carboxy protecting group.

Compound I may be converted to penem antibiotics by known methods, as shown, for example, in U.S. Patent Nos. 4,503,064; 4,530,753; and 4,559,333.

Compounds of the formula 10 may be prepared by known methods. In the inventive process of the invention, it is preferred that Pr be a hydroxy protecting group, preferably t-butyldimethylsilyl. Preparation of compounds of the formula 10 is disclosed, for example, in U.K. Patent Application No. 2,156,814A (published April 6, 1984). As shown therein, the following reaction scheme may be used:

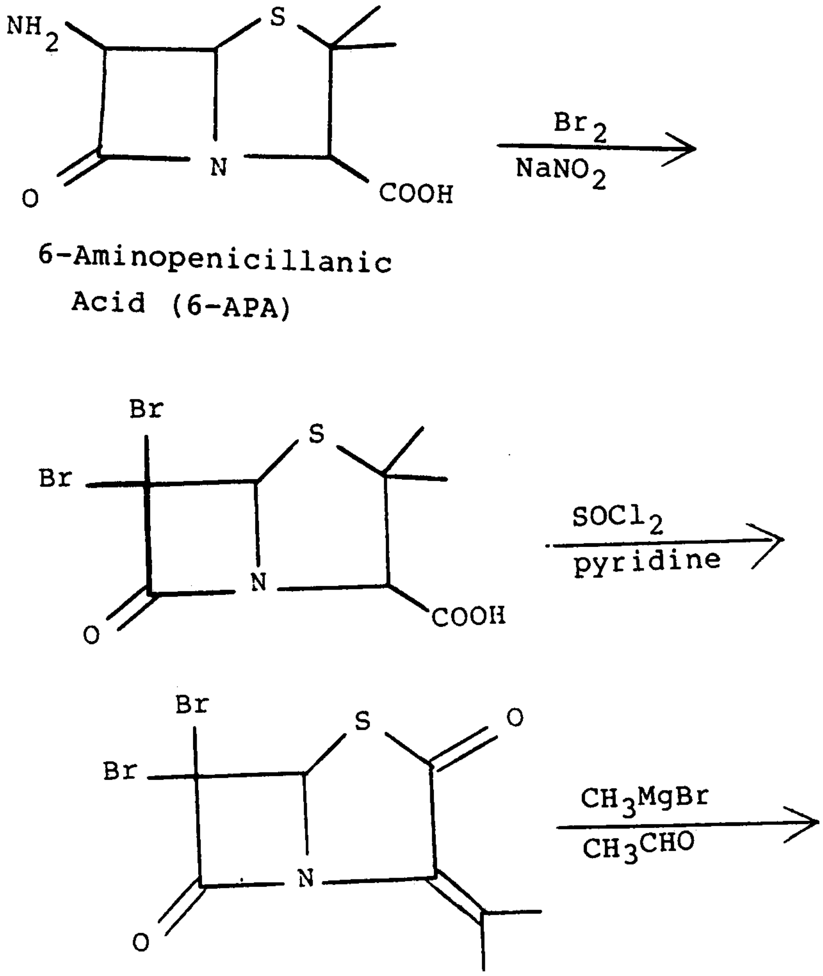


6-Aminopenicillanic Acid (6-APA)

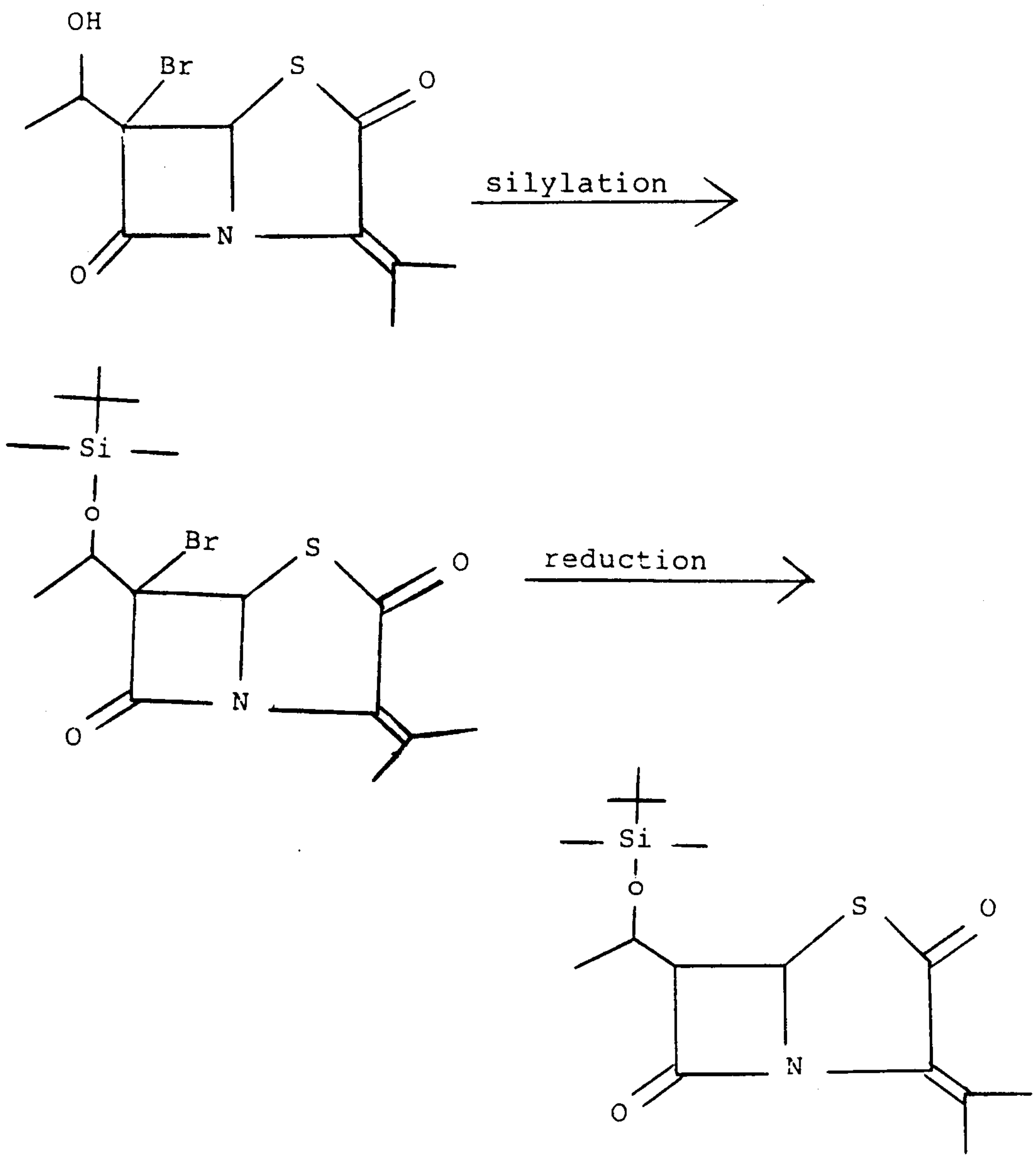


a compound of formula 10
with Pr being t-butyldimethylsilyl

The process of this invention does not require the removal and re-introduction of the sulfur atom which originates with 6-Aminopenicillanic acid. In addition, the process does not require the isolation of all the intermediates and is thus efficient and economical. The process utilizes and provides a means to prepare novel and known intermediates used ultimately in known processes for making penem antibiotics. High yields are achieved with preferred embodiments.

Nomenclature used herein for the various penem and azetidinone compounds is illustrated as follows, with the appropriate numbering system indicated and the stereoisomerism shown.

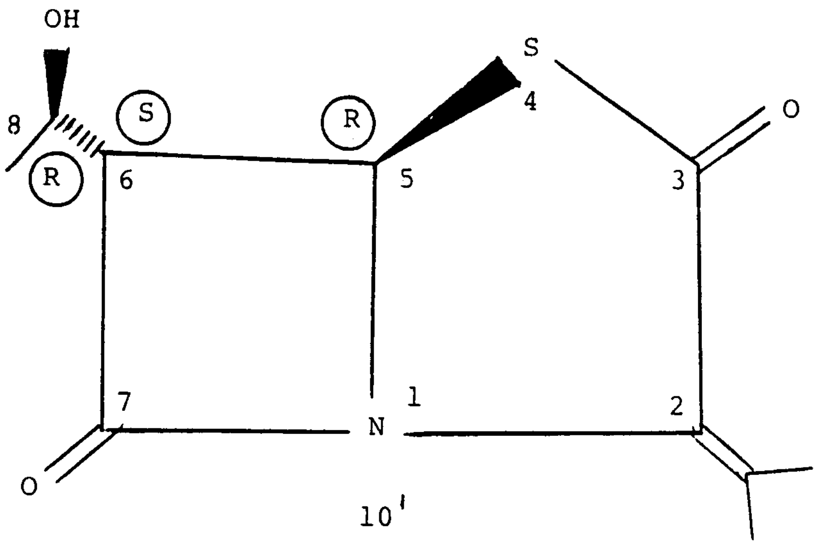

10'

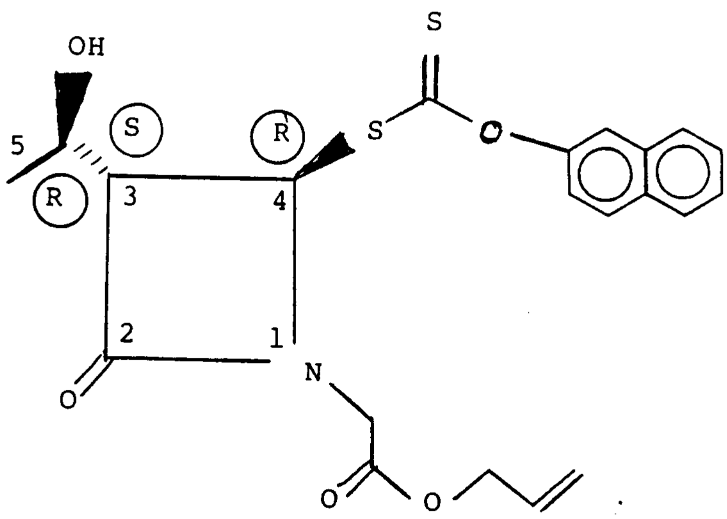

I'

Compound 10' is (5R,6S,8R)-3,7-dioxo-6-(1-hydroxyethyl)-2-(1-methylethylidene)-4-thia-1-azabicyclo[3.2.0.]-heptane; and compound I' is (3S,4R,5R)-1-(allyloxycarbonyl)methyl-3-(1-hydroxyethyl)-4-beta-naphthoxy-(thiocarbonyl)thio-2-azetidinone.

The preferred stereochemistry of the 1-hydroxyethyl side chain on compounds used in and prepared by the process of this invention is R as defined by the Cahn-Ingold-Prelog rules, as indicated by the R below carbon 5 in compound I' and carbon 8 in compound 10', and the remaining chiral centers are indicated by the appropriate R or S.

The inventive process is summarized in the following paragraphs.

In its broadest aspects, the invention comprises a process for producing a compound having the formula

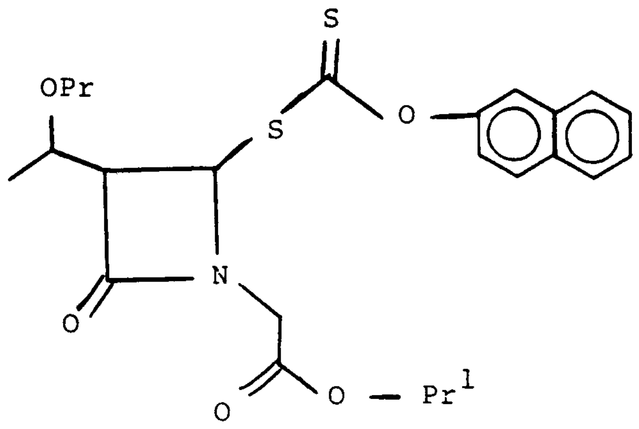

I

wherein Pr is hydrogen or a hydroxy protecting group (preferably t-butyldimethylsilyl) and $Pr^1$ is a carboxy protecting group (preferably allyl) comprising reacting a compound of the formula

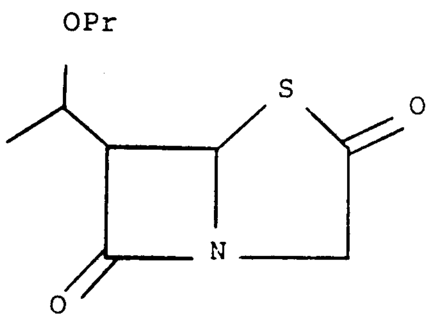

5

wherein Pr is as previously defined, with a compound having the formula $Pr^1$-OH, wherein $Pr^1$ is as previously defined, and either mineral acid (preferably hydrochloric acid) or a silver salt of an organic base (preferably silver imidazolate), and reacting the product so produced with a compound having the formula:

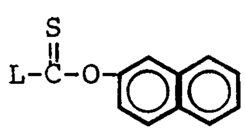

wherein L is a leaving group (preferably chlorine).

Compound 5 may be produced as follows

(a) reacting a compound having the formula

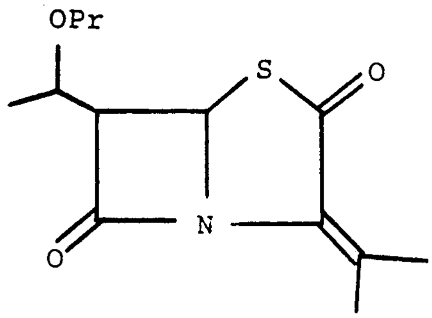

10

wherein Pr is hydrogen or a hydroxy protecting group (preferably t-butyldimethyl-silyl) with ozone, and

(b) reacting the product of step (a) with a compound having the formula $P(OR^1)_3$, wherein $R^1$ is loweralkyl

(preferably ethyl), arylloweralkyl or benzyl, and adding water to the reaction mixture to produce the compound of formula 5.

In the above processes, it is preferred that compounds 5 and 10 have the stereochemistry 5R,6S,8R and that compound I has the stereochemistry 3S,4R,5R.

Novel compounds produced and used by this invention and which are a part thereof are as follows:

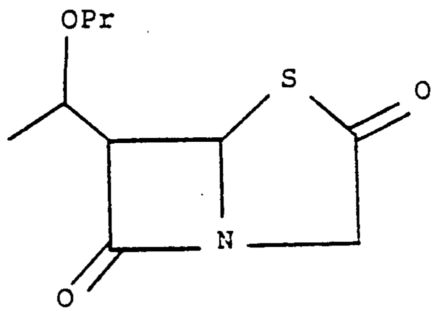

5

wherein Pr is hydrogen or a hydroxy protecting group (preferably t-butyldimethylsilyl), and

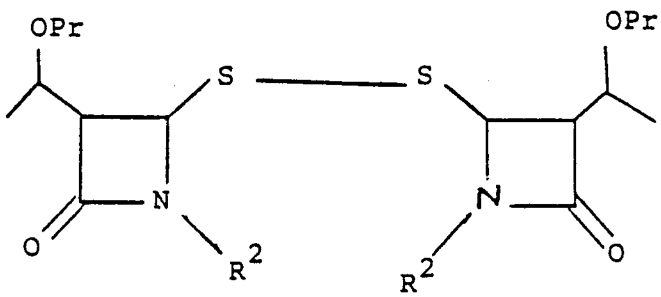

20

wherein both Pr groups are either hydrogen or a hydroxy protecting group (preferably t-butyldimethylsilyl) and each $R^2$ is the same and is

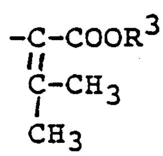
,
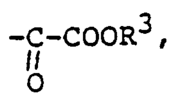
,

or

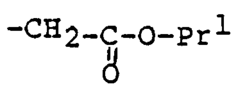

wherein $R^3$ is loweralkyl (preferably methyl) and $Pr^1$ is a carboxy protecting group (preferably allyl).

The inventive process will now be described in detail. In this description, preferred embodiments of the processes are illustrated. However, those skilled in chemistry will recognize that other embodiments, limited only by the appended claims, are operable.

One embodiment of the inventive process is shown in reaction scheme A:

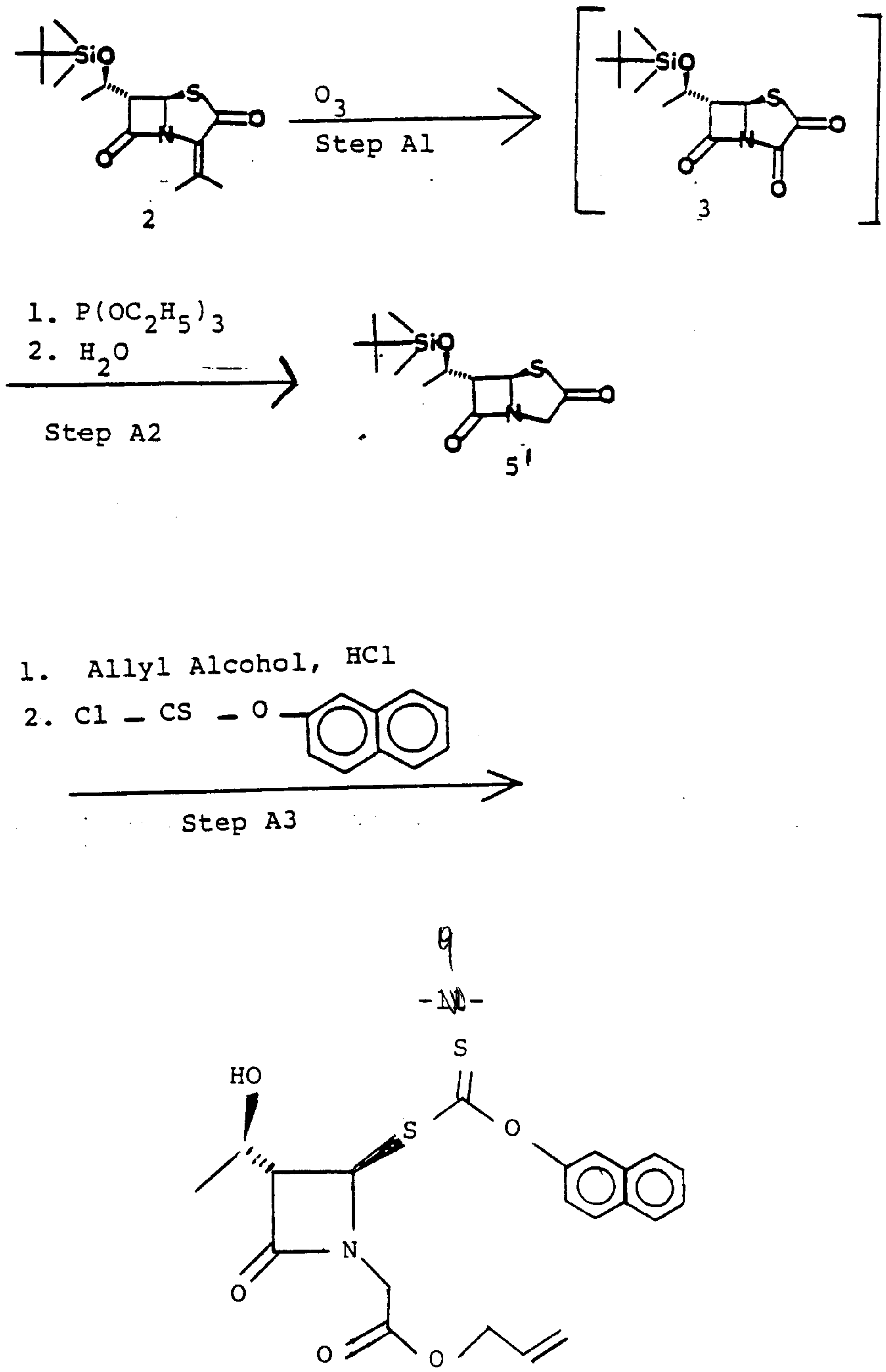


2

3

5'

I'

In Step A1, compound 2, produced as shown in U.K. 2,156,814A, is treated with ozone in an anhydrous inert, organic solvent, e.g., dry acetone, at atmospheric pressure at about -50 to -84°C preferably about -78°C under an inert atmosphere, e.g. nitrogen, until the reaction is complete as evidenced by the formation of a blue colored reaction solution. The resulting compound 3 is not isolated but is used in the next step of the reaction, Step A2.

In Step A2, compound 3 from Step A1 is treated with a compound having the formula $P(OR^1)_3$, wherein $R^1$ is loweralkyl, arylloweralkyl, or benzyl. The compound $P(OR^1)_3$ is preferably triethylphosphite. The reaction mixture is allowed to warm to room temperature (about 20-25°C) and then treated with water until

the reaction is complete, about 30 minutes, and the product, compound 5′, is recovered as a white solid.

In Step A3, compound 5′ is converted to compound I′ in a two step reaction, first, by reaction with a compound having the formula $Pr^1$-OH (preferably allyl alcohol, as shown) in concentrated mineral acid (preferably HCl) at room temperature until the reaction is complete, e.g. about 30 hours, and second, by reaction of the resulting product in an inert organic solvent, e.g. methylene chloride, at about -10 ° C to +10 ° C, preferably 0 ° C, with a compound having the formula

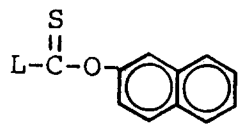

wherein L is a leaving group (preferably chlorine as shown in reaction scheme A), with or without an organic nitrogen base or other acid scavenger, then recovering the product, compound I′.

Reaction Scheme A usually produces a compound of formula I wherein Pr is hydrogen. Since the hydroxy group in Compound I is usually protected when compound I is used for further synthesis, it is more preferable to use reaction scheme B, which provides higher yields.

Reaction Scheme B:

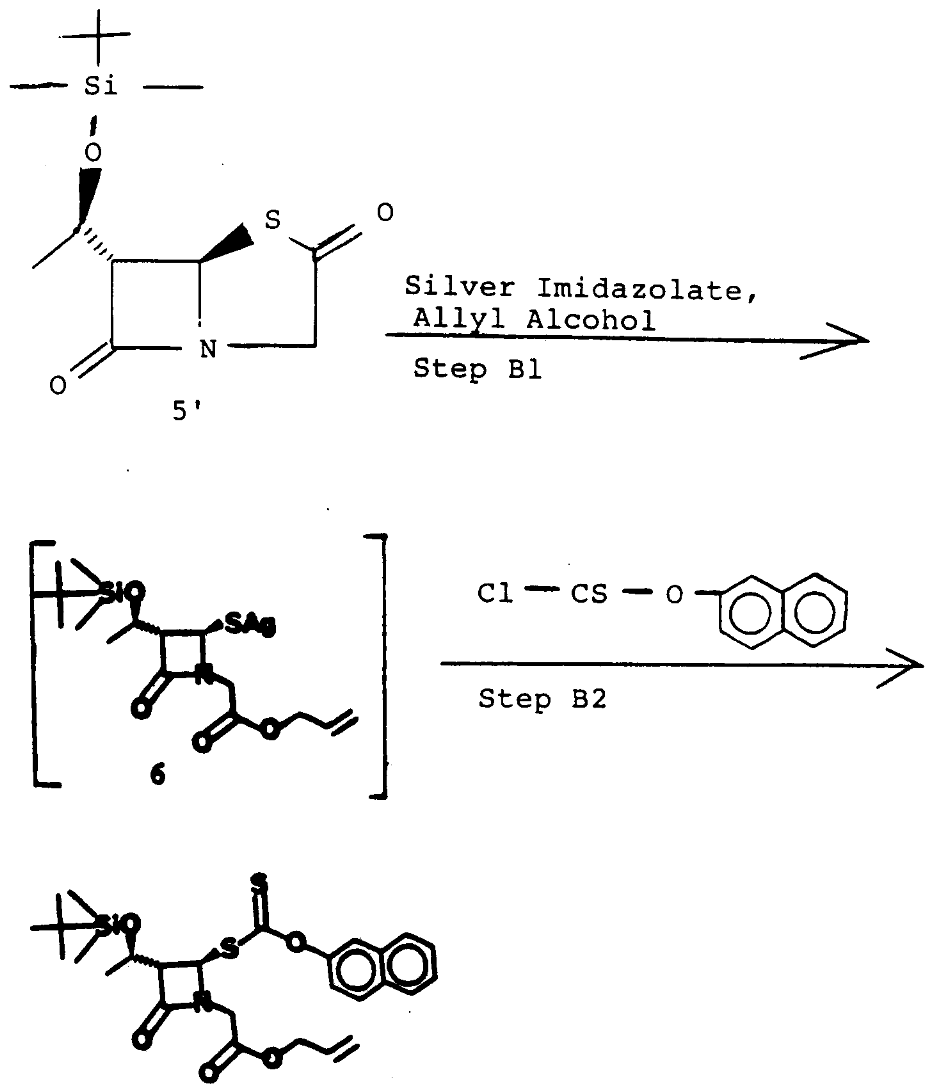


5'

6

I"

In Step B1 compound 5' is reacted with a compound having the formula $Pr^1$-OH (preferably allyl

alcohol, as shown) and a silver salt of an organic base (preferably silver imidazolate, as shown) at room temperature until the reaction is completed in about 30 hours. The resulting product, compound 6, need not be isolated for use in the following Step B2.

In Step B2, compound 6 is converted to compound I" by reaction in an inert organic solvent, e.g., methylene chloride, with a compound of the formula

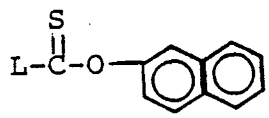

wherein L is a leaving group, (preferably chlorine, as shown in reaction scheme B) at room temperature until the reaction is completed in about one hour. The product, compound I" is then recovered in high yield.

As used herein "hydroxy protecting group" means any group conventionally used for this purpose, the only requirements being compatibility during protection and deprotection reactions with conventional reagents for this purpose which will not adversely affect the structure of the compounds. Typical of such groups are those listed in Green, "Protecting Groups in Organic Synthesis" John Wiley and Sons, New York, NY (1981). Most preferred for use in this invention is tertiarybutyldimethylsilyl. Others which are very suitable for use in this invention are 2,2,2-trichloroethoxycarbonyl, acetate, 1-ethoxyethyl and isoamyldimethylsilyl.

"Carboxy protecting group" means conventional carboxy protectors such as allyl, p-nitrobenzyl, benzyl or benzyhydryl, with allyl preferred.

A "suitable inert organic solvent" means any organic solvent or combination of solvents that is unreactive in the reaction being conducted and is a solvent for the reactants. Such solvents used in the various reactions of this invention are identified in the discussion of the reaction schemes and in the examples.

"Mineral acid" means inorganic acids such as hydrochloric acid, nitric acid, sulfuric acid and phosphoric acid.

"Lower alkyl" refers to straight or branched chain alkyl groups having 1 to 6 carbon atoms. Of course this definition also applies to loweralkyl groups included with other groups as in lower alkanol.

The term aryl includes phenyl and naphthyl. All of the groups phenyl, aryl, and lower alkyl may be substituted with substituents that promote or do not prevent the desired reactions.

The following examples describe the process of the present invention. Throughout these examples "NMR" denotes nuclear magnetic resonance spectra, the spectra described, although in some cases incomplete, are sufficient to identify the compound involved; "mp" means melting point; "HPLC" means high pressure liquid chromatography; "ether" means diethylether; and the boiling range of the petroleum ether (pet ether) is 35 ° C-60 ° C. Flash chromatography on silica gel follows the procedure of Still, et al., J. Organ. Chem., 43, 2923 (1978).

Example 1 illustrates steps A1 and A2 to produce compound 5′.

Example 1

(5R,6S,8R)-6-[1-(t-Butyldimethylsilyloxy)ethyl]-3,7-dioxo-4-thia-1-azabicyclo[3.2.0]heptane

Take 2.019 grams (0.0059 moles) (5R,6S,8R)-6-[1-(t-butyldimethylsilyloxy)ethyl]-3,7-dioxo-2-(1-methylethylidene)-4-thia-1-azabicyclo-[3.2.0.]heptane, 25 ml dry acetone and add to a nitrogen-flushed 100 ml 3-necked flask. Cool to about -78 ° C, bubble ozone through until the solution remains a blue color and stir for 5 minutes, then bubble nitrogen through until the solution is colorless yielding (5R,6S,8R)-6-[1-(t-butyldimethylsilyloxy)ethyl]-4-thia-2,3,7-trioxo-1-azabicyclo[3.2.0]heptane which is not isolated during the reaction but is identified based on its $^{13}C$ NMR spectra.

$^{13}C$ NMR: ($CD_3COCD_3$, BB), $\delta$ = 189.8, 164.2, 156.9, 69.8, 65.0, 50.4, 25.9, 21.7, 18.4, -4.0, -5.5.

Add 3.44 grams (0.0207 moles) freshly distilled triethylphosphite and let warm slowly to room temperature. Add 0.5 ml water after 4 hours, stir for 30 minutes and concentrate using a rotary evaporator. Flash chromatograph the residue on silica gel (5-100% ethyl ether/pet ether) to yield the title product as a white solid.

mp: 65-66 ° C (Recrystallized from ethyl ether/pet ether)

$^1H$ NMR: ($CDCl_3$), $\delta$ = 5.37 (s,1H), 4.37 (d,1H,J = 16.8 Hz), 4.30 (m,1H), 3.53 (dd,1H,J = 1.5,4.4 Hz), 3.44

(dd,1H,J = 0.9,16.8 Hz), 1.27 (d,3H,J = 6.2 Hz), 0.87 (s,9H), 0.08 (s,3H) 0.07 (s,3H).

Example 2 illustrates steps B1 and B2, i.e. the preferred way to convert compound 5′ to a compound of formula I, in this case a compound of formula I″.

Example 2

(3S,4R,5R)-1-(Allyloxycarbonyl)methyl-3-[1-(t-butyldimethylsilyloxy)ethyl]4-β-naphthoxy(thiocarbonyl)thio-2-azetidinone

Take 0.317 grams (0.0011 moles) (5R,6S,8R)-6[1-(t-butyldimethylsilyloxy)ethyl]-3,7-dioxo-4-thia-1-azabicyclo[3.2.0]heptane, 0.063 grams (0.0011 moles) allyl alcohol, 10 ml acetonitrile and add to a nitrogen-flushed 25 ml flask. Then add silver imidazolate (0.187 grams, 0.0011 moles), the flask protected from light with aluminum foil and the reaction mixture stirred at RT for 24 hours. Add another equivalent of allyl alcohol (0.063 grams, 0.0011 moles). After another 24 hours, add 25 ml methylene chloride, 25 ml brine and 10 ml water, separate the layers, extract the aqueous layer with 1 × 25 ml methylene chloride, dry the combined organic layers with $Na_2SO_4$ and concentrate using a rotary evaporator. Dissolve the crude solid in 20 ml methylene chloride, cool to 0 ° C, add 0.623 grams (0.0012 moles) O-2-naphthalenylcarbonochloridothioate and stir the reaction mixture for 24 hours. Remove the solid formed by filtering through a pad of celite, wash the celite pad with 3 × 25 ml portions methylene chloride, then wash the combined organic layers with 1 × 25 ml 5% HCl, 1 × 25 ml $H_2O$, 1 × 25 ml saturated $NaHCO_3$, 1 × 25 ml brine and dry ($MgSO_4$). Flash chromatograph on silica gel (50% ethyl ether/pet ether) the residue obtained after concentration using a rotary evaporator to yield the product.

$^1H$ NMR: ($CDCl_3$), δ = 8.00-7.25 (br m,7H), 5.89 (d,1H,J = 2.6 Hz), 4.28 (d,1H,J = 17.8 Hz), 3.96 (d,1H,J = 17.8 Hz), 3.37 (dd,1H,J = 2.5,6.0 Hz), 1.34 (d,3H,J = 6.1 Hz), 0.91 (s,9H), 0.12 (s,6H).

Example 3 illustrates step A3.

Example 3

(3S,4R,5R)-1-(Allyloxycarbonyl)methyl-3-(1-hydroxyethyl)-4-β-naphthoxy-(thiocarbonyl)thio-2-azetidinone

Take 0.290 grams (0.0010 moles) (5R,6S,8R)-6-[1-(t-butyldimethylsilyloxy)ethyl]-3,7-dioxo-4-thia-1-azabicyclo[3.2.0]heptane, 3 ml allyl alcohol, 2 pipette drops concentrated HCl and add to a nitrogen-flushed 25 ml flask. After 30 hours stirring at room temperature, concentrate on a rotary evaporator, dissolve the residue in 10 ml methylene chloride, cool to 0 ° C and add 0.250 grams (0.0011 moles) O-2-naphthalenylcarbonochloridothioate. After 1 hour stirring at room temperature add 50 ml ethyl ether, 10 ml $H_2O$, separate, extract the aqueous layer with 1 × 25 ml ethyl ether, wash the combined organic layers with 2 × 20 ml 5% HCl, 1 × 20 ml brine, 1 × 20 ml saturated $NaHCO_3$, 1 × 20 ml brine and dry ($MgSO_4$). Concentrate using a rotary evaporator and flash chromatograph the residue on silica gel (15-100% ethyl ether/pet ether) to yield the product.

$^1H$ NMR: ($CDCl_3$), δ = 7.93-7.21 (br m, 7H), 5.96 (d,1H,J = 2.5 Hz), 5.82 (m,1H), 4.37 (d,1H,J = 18.1 Hz), 3.92 (d,1H,J = 18.1 Hz), 3.47 (dd,1H,J = 2.5,5.4 Hz), 2.25 (d,1H,J = 4.2 Hz), 1.41 (d,3H,J = 6.4 Hz).

**Claims**

**Claims for the following Contracting States : BE, CH, DE, FR, GB, GR, IT, LI, LU, NL, SE**

**1.** A process for producing a compound having the formula

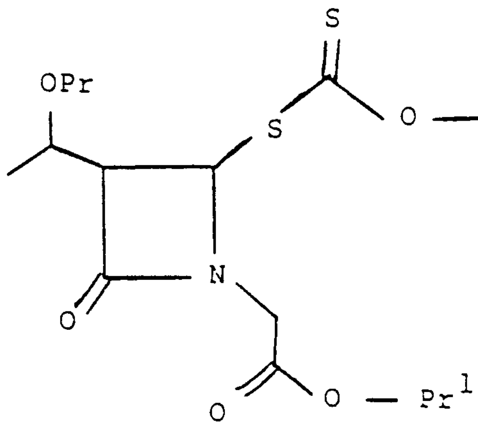

I

wherein Pr is hydrogen or a hydroxy protecting group and $Pr^1$ is a carboxy protecting group, comprising reacting a compound of the formula

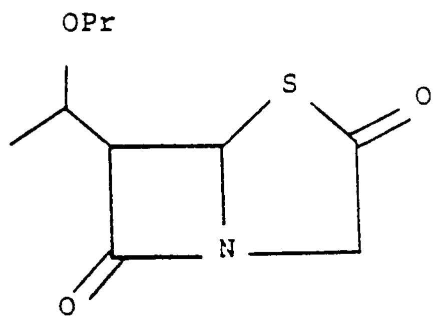

5

wherein Pr is as previously defined, with a compound having the formula $Pr^1$-OH, wherein $Pr^1$ is as previously defined, and either mineral acid or a silver salt of an organic base, and reacting the product so produced with a compound having the formula:

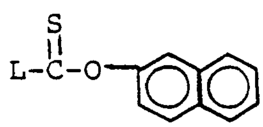

wherein L is a leaving group.

**2.** The process of claim 1 wherein compound 5 is reacted with a compound having the formula $Pr^1$-OH, wherein $Pr^1$ is as defined previously, and mineral acid, and in the compound of formula I produced, Pr is hydrogen.

**3.** The process of claim 1 wherein in compound 5, Pr is a hydroxy protecting group, compound 5 is reacted with a compound having the formula $Pr^1$-OH wherein $Pr^1$ is as defined previously, and a silver salt of an organic base, and in the compound of formula I produced, Pr is a hydroxy protecting group.

**4.** The process of any one of claims 1 to 3 preceded by the following steps:
(a) reacting a compound having the formula

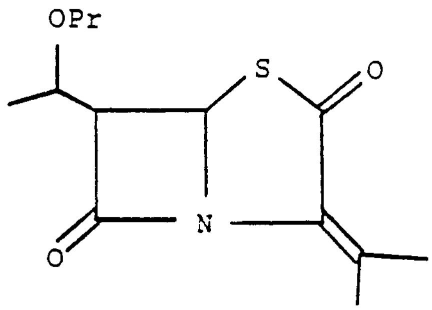

10

wherein Pr is hydrogen or a hydroxy protecting group with ozone, and
(b) reacting the product of step (a) with a compound having the formula $P(OR^1)_3$, wherein $R^1$ is loweralkyl, arylloweralkyl or benzyl, and adding water to the reaction mixture to produce the compound of formula 5.

**5.** The process of any one of claims 1 to 4 wherein $Pr^1$ is allyl and L is chlorine.

**6.** The process of claim 4 wherein $R^1$ is ethyl.

**7.** The process of claim 3 wherein the silver salt of an organic base is silver imidazolate.

**8.** The process of any one of claims 1 to 7 wherein the compound produced has the stereochemistry 3S,4R,5R.

**9.** A compound having the formula

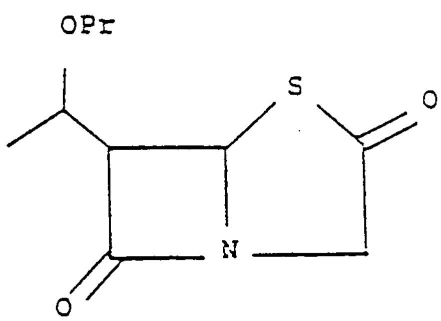

5

wherein Pr is hydrogen or a hydroxy protecting group.

**10.** The compound of claim 9 wherein Pr is t-butyldimethylsilyl.

**Claims for the following Contracting States : AT, ES**

**1.** A process for producing a compound having the formula

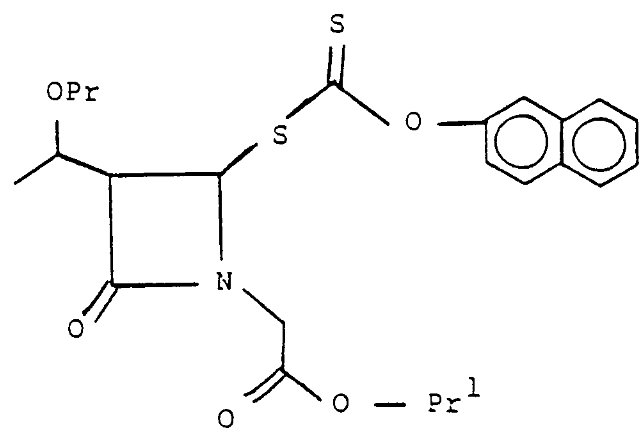

I

wherein Pr is hydrogen or a hydroxy protecting group and $Pr^1$ is a carboxy protecting group, comprising reacting a compound of the formula

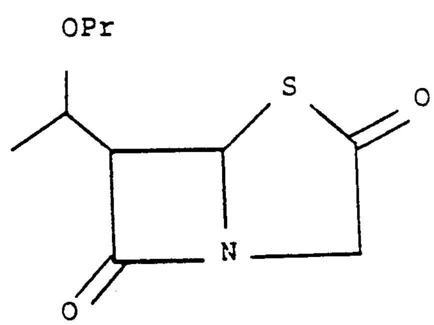

5

wherein Pr is as previously defined, with a compound having the formula $Pr^1$-OH, wherein $Pr^1$ is as previously defined, and either mineral acid or a silver salt of an organic base, and reacting the product so produced with a compound having the formula:

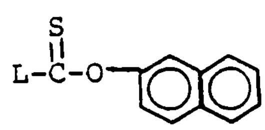

wherein L is a leaving group.

2. The process of claim 1 wherein compound 5 is reacted with a compound having the formula $Pr^1$-OH, wherein $Pr^1$ is as defined previously, and mineral acid, and in the compound of formula I produced, Pr is hydrogen.

3. The process of claim 1 wherein in compound 5, Pr is a hydroxy protecting group, compound 5 is reacted with a compound having the formula $Pr^1$-OH wherein $Pr^1$ is as defined previously, and a silver salt of an organic base, and in the compound of formula I produced, Pr is a hydroxy protecting group.

4. The process of any one of claims 1 to 3 preceded by the following steps:
(a) reacting a compound having the formula

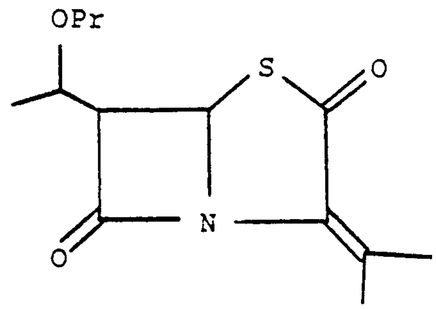

10

wherein Pr is hydrogen or a hydroxy protecting group with ozone, and

(b) reacting the product of step (a) with a compound having the formula $P(OR^1)_3$, wherein $R^1$ is loweralkyl, arylloweralkyl or benzyl, and adding water to the reaction mixture to produce the compound of formula 5.

**5.** The process of any one of claims 1 to 4 wherein $Pr^1$ is allyl and L is chlorine.

**6.** The process of claim 4 wherein $R^1$ is ethyl.

**7.** The process of claim 3 wherein the silver salt of an organic base is silver imidazolate.

**8.** The process of any one of claims 1 to 7 wherein the compound produced has the stereochemistry 3S,4R,5R.

**Revendications**

**Revendications pour les Etats contractants suivants : BE, CH, DE, FR, GB, GR, IT, LI, LU, NL, SE**

**1.** Procédé pour produire un composé ayant la formule :

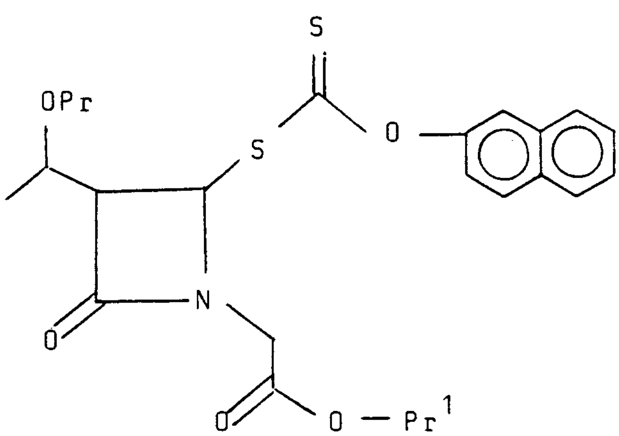

I

où Pr est un atome d'hydrogèneou un groupement protecteur de l'hydroxy et $Pr^1$ est un groupement protecteur du carboxy, comprenant la réaction d'un composé de formule :

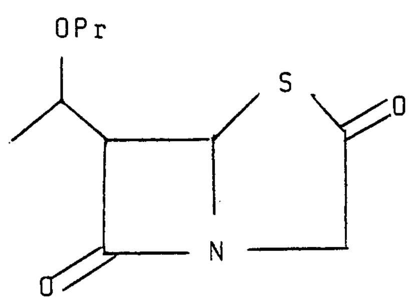

5

où Pr est tel que défini précédemment, avec un composé ayant la formule $Pr^1$-OH, où $Pr^1$ est tel que défini précédemment, et soit un acide minéral, soit un sel d'argent d'une base organique, et la réaction du produit ainsi produit avec un composé ayant la formule :

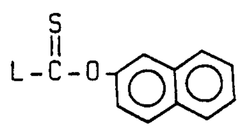

où L est un groupe labile.

2. Procédé selon la revendication 1, où le composé 5 est mis à réagir avec un composé ayant la formule $Pr^1$-OH, où $Pr^1$ est tel que défini précédemment, et un acide minéral, et où dans le composé de formule I produit, Pr est un atome d'hydrogène.

3. Procédé selon la revendication 1, où dans le composé 5, Pr est un groupement protecteur de l'hydroxy, où le composé 5 est mis à réagir avec un composé ayant la formule $Pr^1$-OH où $Pr^1$ est tel que défini précédemment, et un sel d'argent d'une base organique, et où dans le composé de formule I produit, Pr est un groupement protecteur de l'hydroxy.

4. Procédé selon l'une quelconque des revendications 1 à 3, précédé par les étapes suivantes :
(a) réaction d'un composé ayant la formule :

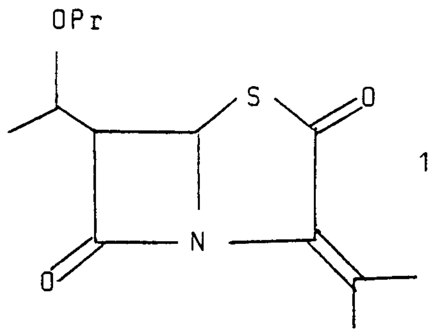

10

où Pr est un atome d'hydrogène ou un groupement protecteur de l'hydroxy avec de l'ozone, et
(b) réaction du produit de l'étape (a) avec un composé ayant la formule $P(OR^1)_3$, où $R^1$ est un alkyle inférieur, un arylalkyle inférieur ou benzyle, et addition d'eau au mélange réactionnel pour produire le composé de formule 5.

5. Procédé selon l'une quelconque des revendications 1 à 4, où $Pr^1$ est allyle et L est un atome de chlore.

6. Procédé selon la revendication 4, où $R^1$ est l'éthyle.

7. Procédé selon la revendication 3, où le sel d'argent d'une base organique est l'imidazolate d'argent.

8. Procédé selon l'une quelconque des revendications 1 à 7, où le composé produit a la stéréochimie 3S,4R,5R.

9. Composé ayant la formule :

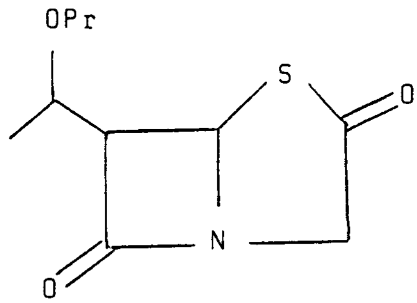

5

où Pr est un atome d'hydrogène ou un groupement protecteur de l'hydroxy.

**10.** Composé selon la revendication 9, où Pr est le t-butyldiméthylsilyle.

**Revendications pour les Etats contractants suivants : AT, ES**

**1.** Procédé pour produire un composé ayant la formule :

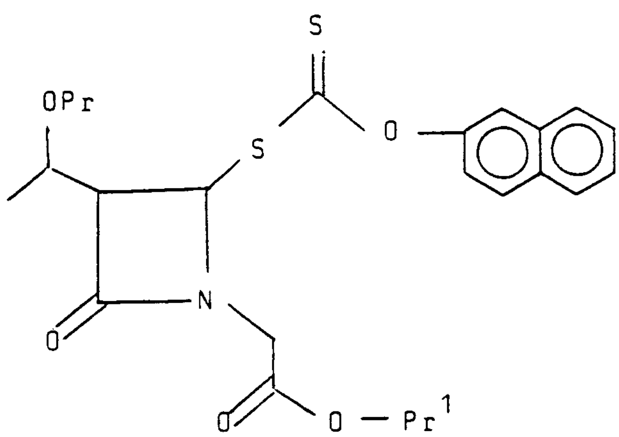

I

où Pr est un atome d'hydrogène d'un groupement protecteur de l'hydroxy et $Pr^1$ est un groupement protecteur du carboxy, comprenant la réaction d'un composé de formule :

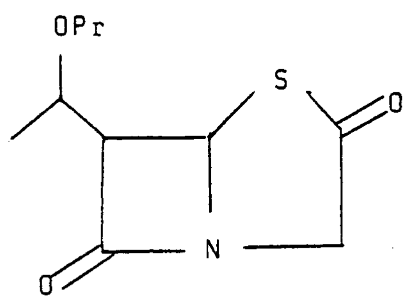

5

où Pr est tel que défini précédemment, avec un composé ayant la formule $Pr^1$-OH, où $Pr^1$ est tel que défini précédemment, et soit un acide minéral, soit un sel d'argent d'une base organique, et la réaction du produit ainsi produit avec un composé ayant la formule :

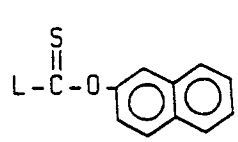

où L est un groupe labile.

**2.** Procédé selon la revendication 1, où le composé 5 est mis à réagir avec un composé ayant la formule $Pr^1$-OH, où $Pr^1$ est tel que défini précédemment, et un acide minéral et où dans le composé de formule I produit, Pr est un atome d'hydrogène.

**3.** Procédé selon la revendication 1, où dans le composé 5, Pr est un groupement protecteur de l'hydroxy, le composé 5 est mis à réagir avec un composé ayant la formule $Pr^1$-OH où $Pr^1$ est tel que défini précédemment, et un sel d'argent d'une base organique, et où dans le composé de formule I produit, Pr est un groupement protecteur de l'hydroxy.

**4.** Procédé selon l'une quelconque des revendications 1 à 3, précédé par les étapes suivantes :
(a) réaction d' un composé ayant la formule :

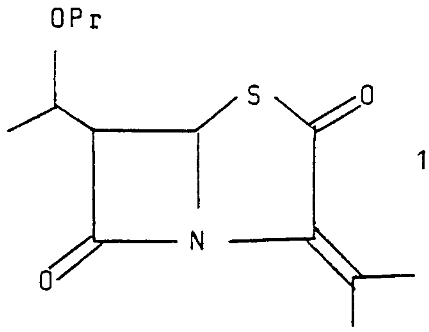

10

où Pr est un atome d'hydrogène ou un groupement protecteur de l'hydroxy avec l'ozone, et
(b) réaction du produit de l'étape (a) avec un composé ayant la formule $P(OR^1)_3$, où $R^1$ est un alkyle inférieur, un arylalkyle inférieur ou benzyle, et addition d'eau au mélange réactionnel pour produire le composé de formule 5.

**5.** Procédé selon l'une quelconque des revendications 1 à 4, où $Pr^1$ est allyle et L est un atome de chlore.

**6.** Procédé selon la revendication 4, où $R^1$ est l'éthyle.

**7.** Procédé selon la revendication 3, où le sel d'argent d'une base organique est l'imidazolate d'argent.

**8.** Procédé selon l'une quelconque des revendications 1 à 7, où le composé produit a la stéréochimie 3S,4R,5R.

**Patentansprüche**
**Patentansprüche für folgende Vertragsstaaten : BE, CH, DE, FR, GB, GR, IT, LI, LU, NL, SE**

**1.** Verfahren zur Herstellung einer Verbindung der Formel

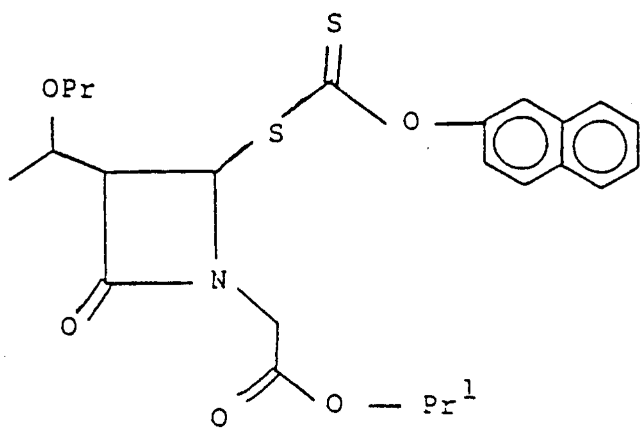

I

worin Pr Wasserstoff oder eine Hydroxy-schützende Gruppe ist, und $Pr^1$ eine Carboxy-schützende Gruppe ist, umfassend die Umsetzung einer Verbindung der Formel

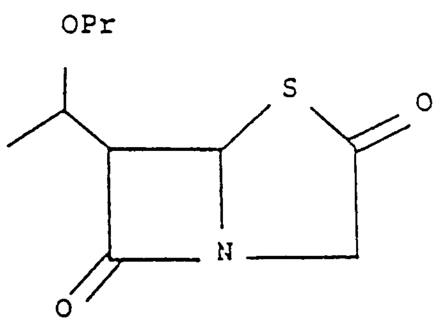

5

worin Pr wie vorstehend definiert ist, mit einer Verbindung der Formel $Pr^1$-OH, worin $Pr^1$ wie vorstehend definiert ist, und entweder Mineralsäure oder einem Silbersalz einer organischen Base, und die Umsetzung des so gebildeten Produkts mit einer Verbindung der Formel:

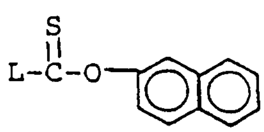

worin L eine abzuspaltende Gruppe ist.

**2.** Verfahren nach Anspruch 1, wobei Verbindung 5 umgesetzt wird mit einer Verbindung der Formel $Pr^1$-OH, worin $Pr^1$ wie vorstehend definiert ist, und Mineralsäure, und in der gebildeten Verbindung der Formel I Pr Wasserstoff ist.

**3.** Verfahren nach Anspruch 1, wobei in Verbindung 5 Pr eine Hydroxyl-schützende Gruppe ist, Verbindung 5 umgesetzt wird mit einer Verbindung der Formel $Pr^1$-OH, worin $Pr^1$ wie vorstehend definiert ist, und einem Silbersalz einer organischen Base, und in der gebildeten Verbindung der Formel I Pr eine Hydroxyl-schützende Gruppe ist.

**4.** Verfahren nach irgendeinem der Ansprüche 1 bis 3, wobei die folgenden Schritte vorgeschaltet sind:
(a) Umsetzung einer Verbindung der Formel

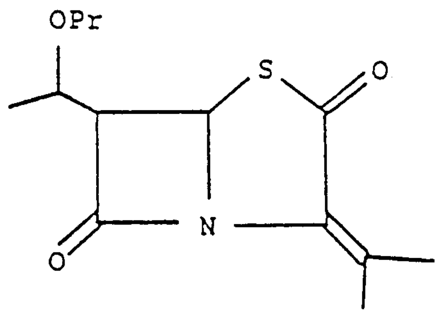

10

worin Pr Wasserstoff oder eine Hydroxyl-schützende Gruppe ist, mit Ozon, und
(b) Umsetzung des Produkts von Schritt (a) mit einer Verbindung der Formel $P(OR^1)_3$, worin $R^1$ niederes Alkyl, Aryl-Niederalkyl oder Benzyl ist, und Zugabe von Wasser zur Reaktionsmischung, um eine Verbindung der Formel 5 zu bilden.

**5.** Verfahren nach irgendeinem der Ansprüche 1 bis 4, wobei $Pr^1$ Allyl und L Chlor ist.

**6.** Verfahren nach Anspruch 4, wobei $R^1$ Ethyl ist.

**7.** Verfahren nach Anspruch 3, wobei das Silbersalz einer organischen Base Silberimidazolat ist.

**8.** Verfahren nach irgendeinem der Ansprüche 1 bis 7, wobei die gebildete Verbindung die Stereochemie 3S,4R,5R hat.

**9.** Verbindung der Formel

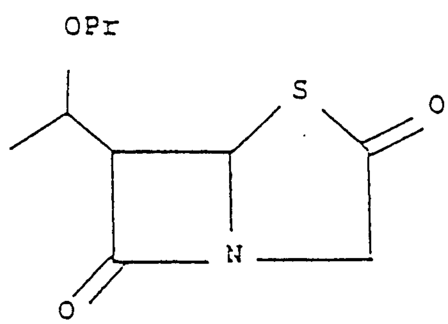

5

worin Pr Wasserstoff oder eine Hydroxy-schützende Gruppe ist.

**10.** Verbindung nach Anspruch 9, worin Pr t-Butyldimethylsilyl ist.

**Patentansprüche für folgende Vertragsstaaten : AT, ES**

**1.** Verfahren zur Herstellung einer Verbindung der Formel

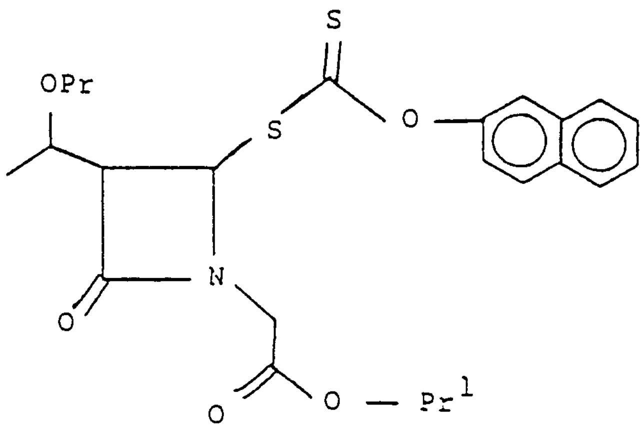

I

worin Pr Wasserstoff oder eine Hydroxy-schützende Gruppe ist, und $Pr^1$ eine Carboxy-schützende Gruppe ist, umfassend die Umsetzung einer Verbindung der Formel

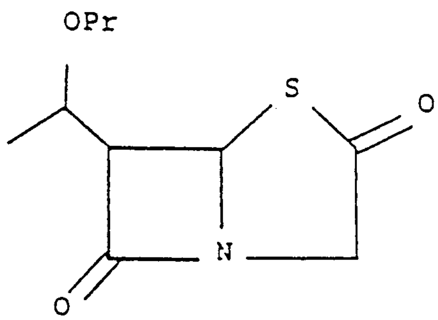

5

worin Pr wie vorstehend definiert ist, mit einer Verbindung der Formel $Pr^1$-OH, worin $Pr^1$ wie vorstehend definiert ist, und entweder Mineralsäure oder einem Silbersalz einer organischen Base, und die Umsetzung des so gebildeten Produkts mit einer Verbindung der Formel:

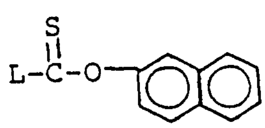

worin L eine abzuspaltende Gruppe ist.

2. Verfahren nach Anspruch 1, wobei Verbindung 5 umgesetzt wird mit einer Verbindung der Formel $Pr^1$-OH, worin $Pr^1$ wie vorstehend definiert ist, und Mineralsäure, und in der gebildeten Verbindung der Formel I Pr Wasserstoff ist.

3. Verfahren nach Anspruch 1, wobei in Verbindung 5 Pr eine Hydroxyl-schützende Gruppe ist, Verbindung 5 umgesetzt wird mit einer Verbindung der Formel $Pr^1$-OH, worin $Pr^1$ wie vorstehend definiert ist, und einem Silbersalz einer organischen Base, und in der gebildeten Verbindung der Formel I Pr eine Hydroxyl-schützende Gruppe ist.

4. Verfahren nach irgendeinem der Ansprüche 1 bis 3, wobei die folgenden Schritte vorgeschaltet sind:
(a) Umsetzung einer Verbindung der Formel

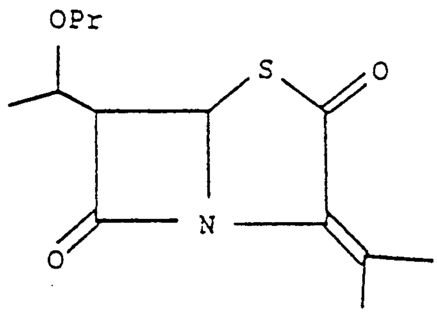

10

worin Pr Wasserstoff oder eine Hydroxyl-schützende Gruppe ist, mit Ozon, und
(b) Umsetzung des Produkts von Schritt (a) mit einer Verbindung der Formel $P(OR^1)_3$, worin $R^1$ niederes Alkyl, Aryl-Niederalkyl oder Benzyl ist, und Zugabe von Wasser zur Reaktionsmischung, um eine Verbindung der Formel 5 zu bilden.

**5.** Verfahren nach irgendeinem der Ansprüche 1 bis 4, wobei $Pr^1$ Allyl und L Chlor ist.

**6.** Verfahren nach Anspruch 4, wobei $R^1$ Ethyl ist.

**7.** Verfahren nach Anspruch 3, wobei das Silbersalz einer organischen Base Silberimidazolat ist.

**8.** Verfahren nach irgendeinem der Ansprüche 1 bis 7, wobei die gebildete Verbindung die Stereochemie 3S,4R,5R hat.